# EUROPÄISCHE PATENTANMELDUNG

(11) **EP 1 710 229 A2**
(43) Veröffentlichungstag der Anmeldung: **11.10.2006**
(21) Anmeldenummer: 06007183.4
(22) Anmeldetag: 05.04.2006
(51) Int. Cl.: C07C 303/00, C07C 303/22, C07C 309/00

(54) **Verfahren zur Herstellung von Arylsulfonsäureanhydriden**

(30) Priorität: 06.04.2005 DE 102005015675
(71) Anmelder: Saltigo GmbH, 51369 Leverkusen (DE)
(72) Erfinder: Schlummer, Björn, 53115 Bonn (DE); Rampf, Florian, 50733 Köln (DE); Naab, Paul, 42287 Wuppertal (DE); Giffels, Guido, 53177 Bonn (DE); Gotta, Matthias, 51061 Köln (DE)
(74) Vertreter: Wichmann, Birgid

(57) **Zusammenfassung**

Gegenstand der vorliegenden Erfindung ist ein Verfahren zur Herstellung von gegebenenfalls substituierten Arylsulfonsäureanhydriden der allgemeinen Formel (I)

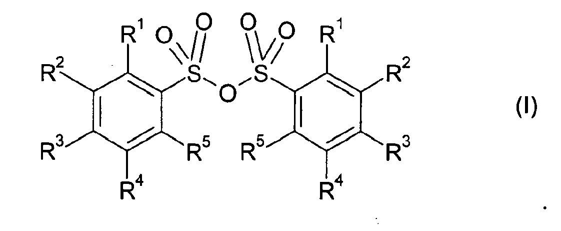

## Beschreibung

Gegenstand der vorliegenden Erfindung ist ein Verfahren zur Herstellung von gegebenenfalls substituierten Arylsulfonsäureanhydriden.

p-Toluolsulfonsäureanhydrid beispielsweise ist ein häufig verwendetes Reagenz zur Darstellung von p-Toluolsulfonatderivaten. Die resultierenden Verbindungen finden vielfältige Anwendung als Intermediate in pharmazeutischen, agrochemischen und weiteren Gebieten der Feinchemikalienchemie. Der Vorteil der Verwendung von p-Toluolsulfonsäureanhydrid gegenüber anderen Reagenzien zur Darstellung von Toluolsulfonaten besteht in den milden Reaktionsbedingungen, insbesondere in dem neutralen pH-Wert während der Reaktion, der z. B. bei der Verwendung von p-Toluolsulfonsäurechlorid nicht gegeben ist.

In der Literatur sind einige Verfahren zur Herstellung beispielsweise von p-Toluolsulfonsäureanhydrid beschrieben.

In J. Am. Chem. Soc. 1952, 74, 394-398 wird die Darstellung durch Reaktion von Phosphorpentoxid, Celite und Asbest mit p-Toluolsulfonsäure bei 125 °C beschrieben. Zur Isolierung des Produktes wird das Reaktionsgemisch aus Benzol/Diethylether umkristallisiert.

Nachteilig bei dieser Reaktionsführung ist die Bildung polymerer Phosphatderivate, die eine großtechnische Durchführung sehr erschweren. In Bull. Chem. Soc. Jpn. 1968, 41, 233 wird die Darstellung durch Reaktion von bis-p-Tolyldisulfan mit Distickstofftetroxid in Tetrachlormethan beschrieben. In J. Am. Chem. Soc. 1954, 76, 1222 ist die Darstellung durch Umsetzung von p-Toluolsulfonsäurechlorid mit Quecksilberoxid in Tetrachlorethan beschrieben. Die Umkristallisation erfolgt aus Benzol/Diethylether. In J. Chem. Soc. C 1968, 1874 ist die Darstellung durch Umsetzung von p-Toluolsulfonsäureiodid mit Kupfer in Tetrachlormethan beschrieben. Der Nachteil dieses Verfahrens ist unter anderem die Verwendung des teuren Iodsalzes. In Chem. Ber. 1965, 98, 3286 ist die Darstellung durch Umsetzung von p-Toluolsulfonsäure mit Phenylcyanat in Benzol beschrieben, wobei ein Nachteil dieses Verfahrens in der Verwendung des teuren Phenylcyanats liegt. In J. Org. Chem. 1963, 28, 2024 wird die Darstellung durch Umsetzung von p-Toluolsulfonsäure mit Diphenylquecksilber und Tributylphosphan in Benzol beschrieben. In Houben Weyl Bd. IX 1955, 553 wird die Darstellung durch Umsetzung von wasserfreier p-Toluolsulfonsäure mit Di-p-tolylcarbodiimid in Benzol beschrieben. Ein Nachteil dieser Methodik ist ebenfalls die Verwendung des teuren Di-p-tolylcarbodiimids. Allen diesen Verfahren gemeinsam ist zudem der Nachteil der Verwendung toxischer, z.T. krebserregender und sicherheitsbedenklicher Reagenzien bzw. Lösungsmittel.

In DE 397311 wird die Darstellung durch Umsetzung von p-Toluolsulfonsäurechlorid mit Natriumchlorid in Eisessig/Wasser bei 125 °C beschrieben. Aufgrund der wässrigen Reaktionsbedingungen ist jedoch eine Hydrolyse des Produktes zu erwarten.

In J. Org. Chem. 1971, 36, 4, 528-531 wird die Darstellung durch Umsetzung von wasserfreier p-Toluolsulfonsäure mit Succinyldichlorid bei 80 °C und anschließendem Anlegen von Hochvakuum sowie Extraktion des Produktes mit Diethylether beschrieben. Das Verfahren ist für eine technische Umsetzung jedoch nicht geeignet.

In US-A 4,115,058 wird die Darstellung durch Reaktion von Toluol mit Schwefeltrioxid in Nitromethan bei 0°C beschrieben. Nachteile dieses Verfahrens sind die Verwendung des hochreaktiven Schwefeltrioxids, des sicherheitstechnisch bedenklichen Nitromethans sowie die Bildung von Stellungsisomeren bei der Reaktion.

In Chem. Ber. 1960, 93, 2736 ist die Darstellung durch Umsetzung von p-Toluolsulfonsäurechlorid mit Silber-p-toluolsulfonat in Dichlormethan über drei Tage beschrieben. Nachteile dieses Verfahrens sind die Verwendung des teuren Silbersalzes sowie die überaus lange Reaktionszeit.

In J. Chem. Soc. Rev. 1954, 1860 ist die Darstellung durch Umsetzung von p-Toluolsulfonsäure mit Methoxyacetylen beschrieben. Der Nachteil hierbei ist die Verwendung des schwer zugänglichen instabilen Methoxyacetylens.

In Tetrahedron Lett. 1998, 39, 19, 2919-2920 wird die Darstellung durch Reaktion von p-Toluolsulfonsäurechlorid mit p-Toluolsulfonsäure in Dichlormethan durch Rühren bei 20 °C beschrieben. Die publizierten Ergebnisse konnten allerdings bei der Nachstellung der Synthese nicht bestätigt werden.

In Houben Weyl Bd. IX 1955, 552 wird die Darstellung durch Umsetzung von wasserfreier p-Toluolsulfonsäure mit Thionylchlorid ohne Lösungsmittel beschrieben. Das Produkt soll aus Eiswasser isoliert werden. Jedoch konnte das beschriebene Verfahren nicht reproduziert werden.

In J. Org. Chem. 1994, 59, 15, 4186-4193 wird die Darstellung durch Umsetzung von p-Toluolsulfonsäure mit Thionylchlorid in Benzol beschrieben. Nachteil dieses Verfahrens ist jedoch wiederum die Verwendung von krebserregendem Benzol.

Es bestand somit weiterhin Bedarf an einer effizienten, auch im großtechnischen Maßstab durchführbaren Synthese von Arylsulfonsäureanhydriden, insbesondere p-Toluolsulfonsäureanhydrid, welches die vorangehend beschriebenen Nachteile nicht aufweist.

Die Aufgabe der vorliegenden Erfindung bestand somit darin, ein solches effizientes Verfahren zur Herstellung von Arylsulfonsäureanhydriden, insbesondere p-Toluolsulfonsäureanhydrid, bereitzustellen, bei welchem die gewünschten Produkte in für eine großtechnische Durchführung ausreichender Ausbeute insbesondere unter Vermeidung von toxischen und krebserregenden oder sicherheitstechnisch bedenklichen Substanzen bzw. Lösungsmitteln erhalten werden können.

Überraschend wurde gefunden, dass die Umsetzung der entsprechenden Arylsulfonsäuren mit Thionylchlorid unter Verwendung von aliphatischen Kohlenwasserstoffen als Lösungsmitteln zu Arylsulfonsäureanhydriden in guten Ausbeuten führt. Dies ist insbesondere unter Berücksichtigung der schlechten Löslichkeiten der Arylsulfonsäuren unerwartet.

Gegenstand der vorliegenden Erfindung ist daher ein Verfahren zur Herstellung von Arylsulfonsäureanhydriden der allgemeinen Formel (I), 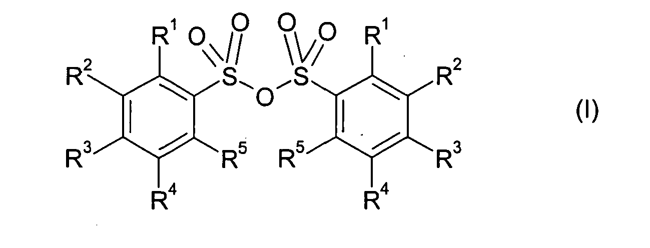 worin
- R¹ bis R⁵: jeweils unabhängig voneinander für Wasserstoff, C₁-C₁₂-Alkyl, bevorzugt C₁-C₆₋Alkyl, C₁-C₁₂-Fluoralkyl, bevorzugt C₁-C₆-Fluoralkyl, C₁-C₁₂-Alkoxy, bevorzugt C₁-C₆-Alkoxy, C₁-C₁₂-Fluoralkoxy, bevorzugt C₁-C₆-Fluoralkoxy, C₄-C₁₈-Aryl, bevorzugt C₅-C₁₀-Aryl, C₅-C₁₉-Arylalkyl, Halogen oder NO₂, stehen,
dadurch gekennzeichnet, dass Arylsulfonsäuren der allgemeinen Formel (II), 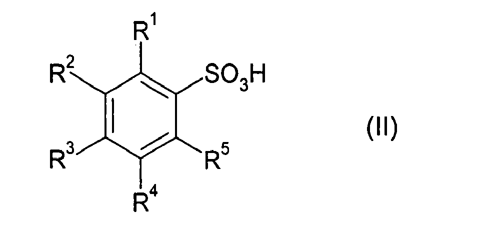 worin R¹ bis R⁵ die für die allgemeine Formel (I) genannte Bedeutung haben,
mit Thionylchlorid in wenigstens einem gegebenenfalls substituierten aliphatischen Kohlenwasserstoff als Lösungsmittel umgesetzt werden.

Als Lösungsmittel können cyclische oder acyclische aliphatische Kohlenwasserstoffe oder Mischungen aus diesen verwendet werden. Beispiele für geeignete cyclische oder acyclische aliphatische Kohlenwasserstoffe sind Cyclopentan, Cyclohexan, Methylcyclohexan, Cycloheptan, Cyclooctan, Hexan, Heptan, Octan und Petrolether. Bevorzugt sind cyclische Kohlenwasserstoffe, besonders bevorzugt sind Cyclopentan, Cyclohexan, Methylcyclohexan oder Mischungen aus diesen.

Thionylchlorid wird bezogen auf die Stoffemenge der eingesetzten Arylsulfonsäure der allgemeinen Formel (II) im Überschuss eingesetzt. Bevorzugt sind Überschüsse von 1,5 bis 5 Äquivalenten, besonders bevorzugt sind Überschüsse von 2 bis 3 Äquivalenten.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

Alkyl beziehungsweise Alkoxy steht jeweils unabhängig für einen geradkettigen, cyclischen, verzweigten oder unverzweigten Alkyl- beziehungsweise Alkoxy-Rest, wobei die genannten Reste weiter substituiert sein können. Gleiches gilt für den Alkylenteil eines Arylalkylrestes.

C₁-C₆-Alkyl steht beispielsweise und bevorzugt für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, C₁-C₁₂-Alkyl darüber hinaus z.B. für n-Heptyl, n-Octyl, n-Decyl und n-Dodecyl.

C₁-C₆-Alkoxy steht beispielsweise und bevorzugt für Methoxy, Ethoxy, n-Propoxy, iso-Propoxy, n-Butoxy, sec.-Butoxy, tert.-Butoxy, n-Pentoxy, cyclo-Hexoxy, cyclo-Pentoxy, n-Hexoxy, C₁-C₁₂₋Alkoxy darüber hinaus z.B. für n-Heptoxy, n-Octoxy, n-Decoxy und n-Dodecoxy.

Fluoralkyl beziehungsweise Fluoralkoxy steht jeweils unabhängig für einen geradkettigen, cyclischen, verzweigten oder unverzweigten Alkyl-Rest beziehungsweise Alkoxy-Rest, der einfach, mehrfach oder vollständig durch Fluoratome substituiert ist.

Beispielsweise steht C₁-C₁₂-Fluoralkyl für Trifluormethyl, 2,2,2-Trifluorethyl, Pentafluorethyl, Nonafluorbutyl, Heptafluorisopropyl, Perfluoroctyl und Perfluordodecyl.

Beispielsweise steht C₁-C₁₂-Fluoralkoxy für Trifluormethoxy, 2,2,2-Trifluorethoxy, Pentafluorethoxy, Nonafluorbutoxy, Heptafluorisopropoxy, Perfluoroctoxy und Perfluordodecoxy.

Aryl steht jeweils für einen mono-, bi- oder tricyclischen heteroaromatischen Rest mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, oder vorzugsweise für einen mono-, bi- oder tricyclischen carbocyclischen aromatischen Rest mit 5 bis 18, bevorzugt 6 bis 10 Gerüstkohlenstoffatomen.

Beispiele für mono-, bi- oder tricyclische carbocyclische aromatische Reste mit 5 bis 18 Gerüstkohlenstoffatomen sind Phenyl, Biphenylyl, Naphtyl, Phenanthrenyl, Anthracenyl oder Fluorenyl, mono-, bi- oder tricyclische heteroaromatische Reste mit 5 bis 18 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuranyl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Halogen, Cyano, C₁-C₁₂-Alkyl, C₁-C₁₂-Fluoralkyl, C₁-C₁₂-Fluoralkoxy oder C₁-C₁₂-Alkoxy.

**Arylalkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann. Ein Beispiel für ArylalkylReste ist Benzyl.

Halogen bedeutet Fluor, Chlor, Brom oder Iod, bevorzugt Fluor, Chlor oder Brom.

Bevorzugt ist das erfindungsgemäße Verfahren zur Herstellung des Arylsulfonsäureanhydrids der allgemeinen Formel (I) geeignet, worin R¹, R², R⁴ und R⁵ für H stehen und R³ für Methyl steht, d.h. zur Herstellung von p-Toluolsulfonsäureanhydrid.

Das erfindungsgemäße Verfahren kann so durchgeführt werden, dass die Arylsulfonsäure der allgemeinen Formel (II) in wenigstens einem Lösungsmittel vorgelegt und das Thionylchlorid zugegeben wird. Alternativ kann auch das Thionylchlorid in wenigstens einem Lösungsmittel vorgelegt und die Arylsulfonsäure der allgemeinen Formel (II) zugegeben werden. Eine weitere Alternative besteht im konzertierten Zusammengeben beider Edukte in wenigstens einem Lösungsmittel. Beliebige Kombinationen aus diesen Alternativen sind möglich.

Gegebenenfalls kann es weiterhin von Vorteil sein, den Reaktionsansatz bei Vorlage von Arylsulfonsäure der allgemeinen Formel (II) vor Zugabe des Thionylchlorids oder bei Vorlage des Thionylchlorids vor Zugabe der Arylsulfonsäure der allgemeinen Formel (II) zu erhitzen. Bevorzugt ist ein Erhitzen auf Temperaturen von 25 bis 100 °C. Besonders bevorzugt sind Temperaturen von 40 bis 80 °C, ganz besonders bevorzugt sind Temperaturen von 45 bis 65 °C.

Gegebenenfalls kann es von Vorteil sein, den Reaktionsansatz nach vollständiger Zu- bzw. Zusammengabe bei der Reaktionstemperatur nachzurühren. Bevorzugt erfolgt ein solches Nachrühren für einen Zeitraum von 0,1 bis 5 h, besonders bevorzugt sind 0,5 bis 3 h, ganz besonders bevorzugt sind 0,5 bis 1 h.

Das überschüssige Thionylchlorid kann gegebenenfalls im Anschluss an die Nachrührzeit aus dem Reaktionsansatz abdestilliert werden. Die Destillation wird bei Sumpftemperaturen von 70 bis 100 °C durchgeführt, bevorzugt sind 75 bis 90 °C, besonders bevorzugt sind 80 bis 85 °C. Die Destillation kann bei Umgebungsdruck (Normaldruck) oder vermindertem Druck durchgeführt werden. In bevorzugten Ausführungsformen wird bei Normaldruck destilliert. Eine Rückführung des abdestillierten Thionylchlorids in den Umsetzungsprozess ist möglich.

Die Isolierung des gewünschten Arylsulfonsäureanhydrids der allgemeinen Formel (I) erfolgt bevorzugt mittels Ausfällen durch Abkühlen des Reaktionsansatzes gegebenenfalls im Anschluss an das Abdestillieren des überschüssigen Thionylchlorids. Nach dem Abkühlen kann es zudem von Vorteil sein, den Reaktionsansatz bei dieser Temperatur nachzurühren. Die Ausfällung erfolgt beispielsweise durch Abkühlen auf +20°C oder wenigstens eine Temperatur unterhalb von +20°C. Bevorzugt wird auf wenigstens eine Temperatur von -10 bis + 20 °C gekühlt, besonders bevorzugt sind Temperaturen von 0 bis 10 °C. Ganz besonders bevorzugt sind 5 °C. Das Nachrühren kann für einen Zeitraum von 0,1 bis 5 h erfolgen, besonders bevorzugt sind 0,5 bis 3 h, ganz besonders bevorzugt ist 1 h.

In einer bevorzugten Variante wird das erfindungsgemäße Verfahren so durchgeführt, dass die Arylsulfonsäure der allgemeinen Formel (II) in wenigstens einem Lösungsmittel vorgelegt, das Reaktionsgemisch geheizt und zu dieser erwärmten Reaktionslösung das Thionylchlorid zugegeben wird. Nach beendeter Zugabe wird der Reaktionsansatz nachgerührt. Anschließend wird überschüssiges Thionylchlorid abdestilliert und das Reaktionsgemisch gekühlt. Das entstandene Arylsulfonsäureanhydrid wird abgesaugt und durch Überleiten von Schutzgas getrocknet.

Der Vorteil des erfindungsgemäßen Verfahrens liegt darin, dass auf das krebserregende Lösungsmittel Benzol sowie auf weitere toxische, teure und/oder sicherheitstechnisch bedenkliche Substanzen verzichtet werden kann und die gewünschten Arylsulfonsäureanhydride der allgemeinen Formel (I) in für die großtechnische Anwendung ausreichend guten Ausbeuten erhalten werden.

Die folgenden Beispiele dienen der beispielhaften Erläuterung der Erfindung und sind nicht als Beschränkung aufzufassen.

### Beispiele

### Beispiel 1: Darstellung von p-Toluolsulfonsäureanhydrid in Methylcyclohexan als Lösungsmittel

50,0 g (260 mmol) p-Toluolsulfonsäure-Monohydrat wurden in 140 g Methylcyclohexan suspendiert und unter Rühren auf 50 °C erwärmt. Dann wurden über einen Zeitraum von 75 Minuten 99,9 g (831 mmol, 3,2 Äquivalente [eq]) Thionylchlorid so zugetropft, dass eine mäßige Gasentwicklung zu beobachten war. Nach beendeter Zugabe wurde 1 h bei 50 °C nachgerührt. Dann wurden bei einer Sumpftemperatur von 96 °C 20,1 g überschüssiges Thionylchlorid abdestilliert. Der Rückstand wurde auf 5 °C gekühlt und 1 h bei dieser Temperatur gerührt. Der ausgefallene Feststoff wurde abfiltriert und mit 50 ml Methylcyclohexan gewaschen. Nach Trocknen im Vakuum wurden 22,2 g (52% d. Th.) p-Toluolsulfonsäureanhydrid isoliert.

Schmelzpunkt: 118-120 °C, Gehalt: 95,6 Gew.-% (¹H-NMR).

### Beispiel 2: Darstellung von p-Toluolsulfonsäureanhydrid in Cyclohexan als Lösungsmittel

150,0 g (789 mmol) p-Toluolsulfonsäure-Monohydrat wurden in 360 g Cyclohexan suspendiert und unter Rühren auf 50 °C erwärmt. Dann wurden über einen Zeitraum von 190 min 240,0 g (1997 mmol, 2,6 eq) Thionylchlorid so zugetropft, dass eine mäßige Gasentwicklung zu beobachten war. Nach beendeter Zugabe wurde 0,5 h bei 50 °C nachgerührt. Dann wurden bei einer Sumpftemperatur von 71-76 °C 80 g überschüssiges Thionylchlorid/Cyclohexan abdestilliert. Der Rückstand wurde auf 5 °C gekühlt und 1 h bei dieser Temperatur gerührt. Der ausgefallene Feststoff wurde abfiltriert und mit 150 ml Cyclohexan gewaschen. Nach Trocknen im Vakuum wurden 70,5 g (53% d. Th.) p-Toluolsulfonsäureanhydrid isoliert.

Schmelzpunkt: 123-124 °C, Gehalt: 96,1 Gew.% (¹H-NMR).

## Patentansprüche

1. Verfahren zur Herstellung von Arylsulfonsäureanhydriden der allgemeinen Formel (I), 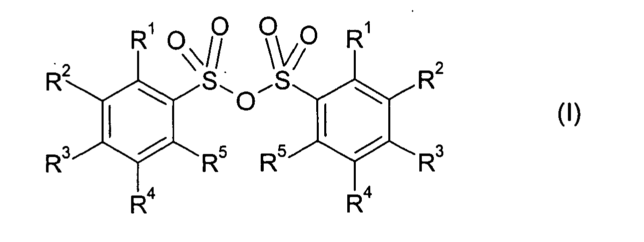 worin
R¹ bis R⁵ jeweils unabhängig voneinander für Wasserstoff, C₁-C₁₂-Alkyl, C₁-C₁₂-Fluoralkyl, C₁-C₁₂-Alkoxy, C₁-C₁₂-Fluoralkoxy, C₄-C₁₈-Aryl oder C₅-C₁₉-Arylalkyl, Halogen, NO₂, stehen,
**dadurch gekennzeichnet, dass** Arylsulfonsäuren der allgemeinen Formel (II), 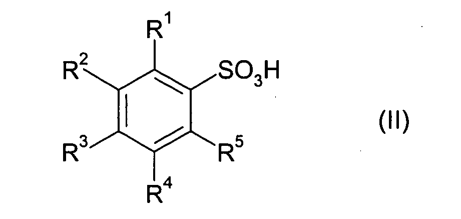 worin R¹ bis R⁵ die für die allgemeine Formel (I) genannte Bedeutung haben,
mit Thionylchlorid in wenigstens einem gegebenenfalls substituierten aliphatischen Kohlenwasserstoff als Lösungsmittel umgesetzt werden.

2. Verfahren gemäß Anspruch 1, **dadurch gekennzeichnet, dass** als Lösungsmittel wenigstens ein gegebenenfalls substituierter cycloaliphatischer Kohlenwasserstoff eingesetzt wird.

3. Verfahren gemäß Anspruch 1 oder 2, **dadurch gekennzeichnet, dass** als Lösungsmittel Cyclopentan, Cyclohexan, Methylcyclohexan oder Mischungen aus diesen eingesetzt werden.

4. Verfahren gemäß wenigstens einem der Ansprüche 1 bis 3, **dadurch gekennzeichnet, dass** R¹, R², R⁴ und R⁵ für H stehen und R³ für Methyl steht.

5. Verfahren gemäß wenigstens einem der Ansprüche 1 bis 4, **dadurch gekennzeichnet, dass** Thionylchlorid bezogen auf die Stoffmenge der Sulfonsäure der allgemeinen Formel (II) im Überschuss eingesetzt wird.

6. Verfahren gemäß wenigstens einem der Ansprüche 1 bis 5, **dadurch gekennzeichnet, dass** die Sulfonsäure der allgemeinen Formel (II) in dem oder den Lösungsmittel(n) vorgelegt, auf Temperaturen von 25 bis 100°C erwärmt und zu dieser erwärmten Reaktionslösung das Thionylchlorid gegeben wird.

7. Verfahren gemäß wenigstens einem der Ansprüche 1 bis 6, **dadurch gekennzeichnet, dass** der Reaktionsansatz nach Zugabe des Thionylchlorids für einen Zeitraum von 0,1 bis 5 h nachgerührt wird.

8. Verfahren gemäß wenigstens einem der Ansprüche 1 bis 7, **dadurch gekennzeichnet, dass** das überschüssige Thionylchlorid gegebenenfalls im Anschluss an die Nachrührzeit aus dem Reaktionsansatz abdestilliert wird.

9. Verfahren gemäß wenigstens einem der Ansprüche 1 bis 8, **dadurch gekennzeichnet, dass** das Arylsulfonsäureanhydrid der allgemeinen Formel (I) durch Abkühlen des Reaktionsansatzes auf +20°C oder wenigstens eine Temperatur unterhalb von +20°C gegebenenfalls im Anschluss an das Abdestillieren des überschüssigen Thionylchlorids ausgefällt wird.
